Europäisches Patentamt

European Patent Office

Office européen des brevets

(19)

(11) Publication number: **0 008 482**
**B1**

(12) **EUROPEAN PATENT SPECIFICATION**

(45) Date of publication of patent specification: **20.04.83**

(21) Application number: **79200455.8**

(22) Date of filing: **15.08.79**

(51) Int. Cl.³: **C 07 C 69/716,**
**C 07 C 67/347,**
**C 07 C 59/185,**
**C 07 C 51/373,**
**C 07 C 121/34,**
**C 07 C 120/00**

(54) Process for the preparation of delta keto-acids and derivatives thereof.

(30) Priority: **19.08.78 NL 7808605**

(43) Date of publication of application:
**05.03.80 Bulletin 80/5**

(45) Publication of the grant of the patent:
**20.04.83 Bulletin 83/16**

(84) Designated Contracting States:
**BE CH DE FR GB IT NL SE**

(56) References cited:
**FR - A - 2 229 679**

(73) Proprietor: **STAMICARBON B.V.**
**Postbus 10**
**NL-6160 MC Geleen (NL)**

(72) Inventor: **Meyer, Peter Johan Nicolaas**
**Burg. Luytenstraat 31**
**NL-6151 GE Munstergeleen (NL)**
Inventor: **Penders, Josef Maria**
**Cuyleborg 141**
**NL-6228 BD Maastricht (NL)**

(74) Representative: **Pinckaers, August René et al,**
**OCTROOIBUREAU DSM Postbus 9**
**NL-6160 MA Geleen (NL)**

Courier Press, Leamington Spa, England.

## Process for the preparation of delta keto-acids and derivatives thereof

The invention relates to a process for the preparation of delta keto-acids or an ester, the nitrile or the amide thereof by reaction of a ketone with an acrylic compound being an acrylic acid or an ester, the nitrile or the amide of an acrylic acid in the liquid phase.

A reaction of this type, which is known by the name of Michael's addition, can give products that are suitable as starting materials for the preparation of valuable products. The delta keto-acid ester obtained upon addition of an acrylic ester to cyclohexanone can, for instance, be converted into dihydrocoumarin, which is important in the scents industry (see United States Patent Specification 3,442,910). Addition of acrylic acid or an acrylic ester to acetone yields a compound that can be converted by cyclization into dihydroresorcinol, which can be dehydrogenated to form resorcinol, a well-known starting material in the plastics industry.

It is commonly known (see, for instance, British Patent Specifications 1,303,949, 1,389,510 and 1,476,153) to use a primary amine or a Schiff base as a catalyst in the addition of a ketone having at least one activated H-atom at the alpha position to an acrylic acid or the ester or the nitrile of such acid. In the French Patent Specification 2,229,679 also other catalyst are disclosed for this addition reaction e.g. a $\beta$-amino-propionate derived from a primary amine. Also a secondary amine, such as piperidine, has been mentioned as a catalyst for some Michael addition reactions (see Adams, organic reactions, vol. 10, p. 415—477, John Wiley and Sons 1959).

A catalyst has now been found that can give better results in said addition reaction.

The process according to the invention for the preparation of delta keto-acids or an ester, the nitrile or the amide thereof by reaction of a ketone having at least one activated H-atom in the alpha position with an acrylic compound which is acrylic acid methacrylic acid or crotonic acid, or an ester nitrile or amide thereof in the liquid phase and with the aid of a catalyst is characterized in that the catalyst used is an addition product of a cyclic secondary amine and an $\alpha$-$\beta$-unsaturated compound.

When a catalyst according to the invention is used, a higher selectivity, both with respect to the ketone and with respect to the acrylic compound, and a lower catalyst consumption can be achieved than would be the case with the use of the known catalysts.

The process according to the invention may be started from various ketones, such as, e.g., acetone, methyl-ethyl ketone, methyl-propyl ketone, diethyl ketone, methyl-isopropyl ketone, cyclopentanone, cyclohexanone, 2-methyl cyclohexanone, and 4-methyl cyclohexanone. The process according to the invention is particularly suitable for the conversion of acetone and cyclohexanone. Various acrylic compounds may be used in the process according to the invention. Of practical importance are, especially, acrylic acid, methacrylic acid, crotonic acid, and also esters, the nitrile and the amide of these acids. Very good results can be obtained with the use of the methyl ester and ethyl ester and the nitrile of said acids. The ratio between the amount of ketone and the amount of acrylic compound may be varied. Theoretically, 1 mole of ketone is required per mole of acrylic compound. In most cases excess ketone is used in the conversion of an aliphatic ketone. An amount of over 10 moles of aliphatic ketone per mole of acrylic compound may be used, but does not lead to any practical advantage. The best ratio of ketone to acrylic compound can readily be determined experimentally in practice.

The catalyst according to the invention may be based on varioius cyclic secondary amines, such as, e.g., pyrrolidine, piperidine, morpholine and piperazine, which amines may have been substituted with an alkyl group of 1—4 C-atoms at one or several C-atoms. Of the above amines, pyrrolidine is very suitable, because not only a high selectivity, but also a satisfactory conversion can be achieved. The $\alpha$-$\beta$-unsaturated compound on which the catalyst is also based may be chosen differently. Very suitable are acrylic acid, methacrylic acid, crotonic acid, esters of these acids, such as the methyl and ethyl esters, and the amide or nitrile of said acids. In practice, the catalyst used by preference is an addition product of the amine and the acrylic compound to be converted. The addition of the $\alpha$-$\beta$-unsaturated compound to the cyclic secondary amine may be effected in a known way, e.g. in the way described in Bull. Soc. Chim. France, 1971, No. 5 p. 1717 ff. and J. Am. Chem. Soc. Vol. 66, 1944 p. 725—731. The amount of catalyst may be varied. An amount of 0.05—0.5 mole of catalyst per mole of acrylic compound to be converted is very satisfactory in practice. If a minor amount of an acid compound is present in addition to the catalyst, it may result in an improvement of the selectivity. Examples of suitable acid compounds are acetic acids, adipic acid, benzoic acid, phenol, caproic acid, hydrochloric acid, phosphoric acid, sulphuric acid and ammonium chloride. For instance, 0.001—0.1 mole of acid compound may be used per mole of catalyst.

The process according to the invention is usually carried out at a temperature of between 130 and 250°C. For, at a temperature of over 250°C, the selectivity of the reaction becomes lower, while the reaction proceeds too slowly at temperatures of below 130°C. Temperatures of between 170 and 230°C are particularly suitable. The pressure is not critical and may be varied. Of course, the pressure must be chosen so that the reaction can take place in the liquid phase, whether or not in the presence of a solvent or vehicle.

The ketone and/or the acrylic compound may be converted fully or partly in the process according to the invention. Preferably 20—90% of the acrylic compound is converted. For, a conversion of over 90% may have an adverse effect on the selectivity of the reaction, while a conversion of below 20% results in too large a recirculation of unconverted starting product. After the desired conversion has been reached, the reaction mixture can be separated by distillation, in which, besides the desired product, a fraction rich in catalyst can be separated off which may be re-used.

The invention will be further elucidated in the following examples.

Example I

290.0 grams of acetone, 86.0 grams of methyl acrylate, 19.6 grams of methyl-β-pyrrolidin-1-yl propionate (addition product of pyrrolidine and methyl acrylate) and 0.2 grams of benzoic acid are put together in a 1-litre autoclave. The mixture in the autoclave is heated to 170°C under autogenous pressure and kept at this temperature for 3 hours. The reaction mixture is then cooled and transferred to a distillation flask. The mixture is separated into 316.2 grams of distillate boiling below 132°C and 79.6 grams of residue by distillation at atmospheric pressure. Gas-chromatographic analysis shows that the distillate contains 263.4 grams of acetone and 49.6 grams of methylacrylate. According to gas-chromatographic analysis, the residue contains 18.9 grams of methyl-β-pyrrolidin-1-yl propionate and 58.2 grams of methyl ester of delta-oxocaproic acid. The residue is separated by fractional distillation into a first-running fraction of 20.8 grams with a boiling point of 112—114°C at 5.3 kPa and a main fraction of 56.5 grams with a boiling point of 121.5—122.5°C at 5.3 kPa. According to gas-chromatographic analysis, the first-running fraction contains 89.7% of methyl-β-pyrrolidin-1-yl propionate ($n_D^{23}$=1.4514) and the main fraction 99.8% of 4-oxopentane-carboxylic methyl ester ($n_D^{23}$=1.4270).

42.3% of the total amount of methyl acrylate has been converted. The yield is 88.0% with respect to the amount of acetone consumed and 95.4% with respect to the amount of methyl acetylate converted.

In order to prepare the above addition product, 0.12 mole of pyrrolidine is added, at room temperature, to a solution of 0.12 mole of methyl acrylate in 40 millilitres of ether, after which the mixture is heated with reflux cooling for 24 hours. The ether is then evaporated and the residue is distilled at 2.6 kPa.

Comparative example

Example I is repeated on the understanding that 8.9 grams of pyrrolidine are added instead of 19.6 grams of methyl-β-pyrrolidin-1-yl pro-

pionate. After three hours' reaction at 170°C under autogenous pressure the reaction mixture contains 90.9 grams of methyl ester of delta-oxocaproic acid and 7.3 grams of methyl acrylate according to gas-chromatographic analysis. The yield is 69.0% with respect to converted methyl acrylate and 65.2% with respect to acetone.

Example II

34.8 grams of acrylonitrile, 292.4 grams of acetone, 9.4 grams of β-pyrrolidin-1-yl propionitrile (addition product of pyrrolidine and acrylonitrile) and 0.06 gram of acetic acid are put together in a 1-litre autoclave. The mixture is heated at 200°C for 4 hours under autogenous pressure. After cooling, the mixture is analysed gas-chromatographically. According to the analysis, the reaction mixture contains 9.0 grams of acrylonitrile, 238.4 grams of acetone and 47.1 gram of delta-oxocapronitrile. This means an acrylonitrile conversion of 74.1%. The yield of delta-oxocapronitrile is 82.0% with respect to converted acetone and 87.4% with respect to converted acrylonitrile.

Example III

245.0 grams of cyclohexanone, 244.6 grams of methyl acrylate, 30.4 grams of methyl-β-pyrrolidin-1-yl propionate and 0.1 grams of benzoic acid are put together in a 1-litre autoclave. The mixture in the autoclave is heated to 170°C under autogenous pressure and kept at this temperature for 2 hours. The reaction mixture is then cooled and analysed gas-chromatographically. 40.4 grams of methyl acrylate are detected, while 439.0 grams of methyl-3-(2-oxocyclohexyl)propionate have formed.

The yield of methyl-3-(2-oxocyclohexyl)propionate with respect to the amount of methyl acrylate converted is quantitative and is 99.2% with respect to the amount of cyclohexanone converted.

Example IV

290.0 grams of acetone, 82.8 grams of methyl acrylate, 23.8 grams of methyl-β-piperidin-1-yl propionate (addition product of piperidine and methyl acrylate) and 0.7 gram of benzoic acid are put together in a 1-litre autoclave. The mixture in the autoclave is heated to 210°C under autogeneous pressure and kept at this temperature for 4 hours. After cooling, the reaction mixture is analysed gas-chromatographically 66.6 grams of methyl acrylate are detected, while 24.2 grams of delta-oxocaproic acid methyl ester have formed. The yield of delta-oxocaproic methyl ester is consequently 89.1% with respect to converted methyl acrylate and 92.0% with respect to the amount of acetone converted.

Example V

290.0 grams of acetone, 83.5 grams of methyl acrylate, 16.8 grams of β-pyrrolidin-1-yl propionitrile (addition product of pyrrolidine and acrylonitrile) and 0.6 gram of benzoic acid are put together in a 1-litre autoclave. The mixture in the autclave is heated to 200°C under autogenous pressure and kept at this temperature for 2 hours. After cooling, the mixture is analysed gas-chromatographically. 65.5 grams of methyl acrylate are detected, while 24.2 grams of delta-oxocaproic acid methyl ester have formed. Hence, the yield of delta-oxomethyl caproate is 90.1% with respect to the amount of methyl acrylate converted and 78.0% with respect to the amount of acetone converted.

## Claims

1. Process for the preparation of delta-ketoacids or an ester, the nitrile or the amide thereof by reaction of a ketone having at least one activated H-atom in the alpha position with an acrylic compound which is acrylic acid methacrylic acid, or crotonic acid or an ester, nitrile or amide thereof in the liquid phase and with the aid of a catalyst, characterized in that the catalyst used is an addition product of a cyclic secondary amine and an $\alpha$-$\beta$-unsaturated compound.

2. Process according to claim 1, characterized in that the cyclic secondary amine used is pyrrolidine.

3. Process according to claim 1 or 2, characterized in that the catalyst is an addition product of the amine and an acrylic compound which is the same as that to be converted.

4. Process according to any one of the claims 1—3, characterized in that 0.05—0.5 mole of catalyst is used per mole of acrylic compound to be converted.

5. Process according to any one of the claims 1—4, characterized in that the reaction mixture contains a catalytic amount of an acid compound.

6. Process according to any one of the claims 1—5, characterized in that the reaction is effected at a temperature of 170—230°C.

7. Process according to any one of the claims 1—6, characterized in that the reaction conditions are maintained until 20—90% of the acrylic compound has been converted.

8. Process according to any of the claims 1—7, characterized in that the ketone is acetone or cyclohexanone.

9. Process according to any one of the claims 1—8, characterized in that the acrylic compound is the nitrile, the methyl ester or ethyl ester of acrylic acid, methacrylic acid or crotonic acid.

## Patentansprüche

1. Verfahren zum Herstellen von Delta-Ketosäuren oder eines Esters, von dessen Nitril oder dessen Amid durch eine Reaktion eines Ketons mit mindestens einem aktivierten H-Atom in der Alphastellung mit einer Acrylverbindung, wie Acrylsäure, Methacrylsäure oder Crotonsäure, oder einem Ester, Nitril oder Amid davon in der Flüssigphase und mit Hilfe eines Katalysators, dadurch gekennzeichnet, dass der Katalysator ein Additionsprodukt eines zyklisch sekundären Amins und einer $\alpha$-$\beta$-ungesättigten Verbindung ist.

2. Verfahren nach Anspruch 1, dadurch gekennzeichnet, dass das verwendete zyklisch sekundäre Amin Pyrrolidin ist.

3. Verfahren nach Anspruch 1 oder 2, dadurch gekennzeichnet, dass der Katalysator ein Additionsprodukt des Amins und einer Acrylverbindung ist, die der umzuwandelnden entspricht.

4. Verfahren nach einem der Ansprüche 1— 3, dadurch gekennzeichnet, dass 0,05—0,05 Mol Katalysator je Mol umzuwandelnde Acrylverbindung benutzt werden.

5. Verfahren nach einem der Ansprüche 1— 4, dadurch gekennzeichnet, dass das Reaktionsgemisch eine Katalytische Menge einer. Säureverbindung enthält.

6. Verfahren nach einem der Ansprüche 1— 5, dadurch gekennzeichnet, dass die Reaktion bei einer Temperatur von 170—230°C stattfindet.

7. Verfahren nach einem der Ansprüche 1— 6, dadurch gekennzeichnet, dass die Reaktionsbedingungen aufrechterhalten werden, bis 20— 90% der Acrylverbindung umgewandelt sind.

8. Verfahren nach einem der Ansprüche 1— 7, dadurch gekennzeichnet, dass das Keton Aceton oder cyclohexanon ist.

9. Verfahren nach einem der Ansprüche 1— 8, dadurch gekennzeichnet, dass die Acrylverbindung das Nitril, der Methylester oder Äthylester von Acrylsäure, Methacrylsäure oder Crotonsäure ist.

## Revendications

1. Procédé de préparation d'acides deltacétoniques ou d'un ester, du nitrile ou de l'amide d'un tel acide par réaction d'une cétone contenant au moins un atome d'hydrogène activé dans la position alpha avec un composé acrylique qui consiste en l'acide acrylique, l'acide méthacrylique ou l'acide crotonique ou un ester, nitrile ou amide d'un tel acide, en phase liquide, et à l'aide d'un catalyseur, caractérisé en ce que le catalyseur utilisé est un produit d'addition d'une amine cyclique secondaire et d'un composé alpha, bêta-insaturé.

2. Procédé selon la revendication 1, caractérisé en ce que l'amine cyclique secondaire utilisée est la pyrrolidine.

3. Procédé selon la revendication 1 ou 2, caractérisé en ce que le catalyseur est un produit d'addition de l'amine et d'un composé acrylique identique à celui soumis à la conversion.

4. Procédé selon l'une quelconque des revendications 1 à 3, caractérisé en ce que l'on utilise de 0,05 à 0,5 mole de catalyseur par mole du composé acrylique à convertir.

5. Procédé selon l'une quelconque des revendications 1 à 4, caractérisé en ce que le mélange de réaction contient une quantité catalytique d'un composé acide.

6. Procédé selon l'une quelconque des revendications 1 à 5, caractérisé en ce que la réaction est effectuée à une température de 170 à 230°C.

7. Procédé selon l'une quelconque des revendications 1 à 6, caractérisé en ce que l'on maintient les conditions de réaction jusqu'à ce que l'on ait converti de 20 à 90% du composé acrylique.

8. Procédé selon l'une quelconque des revendications 1 à 7, caractérisé en ce que la cétone est l'acétone ou la cyclohexanone.

9. Procédé selon l'une quelconque des revendications 1 à 8, caractérisé en ce que le composé acrylique est le nitrile, l'ester méthylique ou l'ester éthylique de l'acide acylique, de l'acide méthacrylique ou de l'acide crotonique.